Europäisches Patentamt

European Patent Office

Office européen des brevets

(11) Veröffentlichungsnummer : **0 207 100 B1**

(12) **EUROPÄISCHE PATENTSCHRIFT**

(45) Veröffentlichungstag der Patentschrift :
19.06.91 Patentblatt 91/25

(21) Anmeldenummer : 86900006.7

(22) Anmeldetag : 23.12.85

(86) Internationale Anmeldenummer :
PCT/AT85/00060

(87) Internationale Veröffentlichungsnummer :
WO 86/03685 03.07.86 Gazette 86/14

(51) Int. Cl.⁵ : **B01D 67/00, B01D 71/14, B01D 61/14, B01J 13/02, B01D 53/22, B01J 47/00, B01D 15/08, A61K 9/64, A61K 47/00, C12N 11/04**

(54) VERFAHREN ZUM ÄNDERN DER WIRKSAMEN PORENWEITE EINER STRUKTUR.

Die Akte enthält technische Angaben, die nach dem Eingang der Anmeldung eingereicht wurden und die nicht in dieser Patentschrift enthalten sind.

(30) Priorität : 21.12.84 AT 4069/84
06.03.85 ZA 851706
08.11.85 AT 3247/85

(43) Veröffentlichungstag der Anmeldung :
07.01.87 Patentblatt 87/02

(45) Bekanntmachung des Hinweises auf die Patenterteilung :
19.06.91 Patentblatt 91/25

(84) Benannte Vertragsstaaten :
AT BE CH DE FR GB IT LI LU NL SE

(56) Entgegenhaltungen :
EP-A- 0 126 537
EP-A- 0 154 620
FR-A- 2 230 722
US-A- 3 736 204
US-A- 4 327 710

(56) Entgegenhaltungen :
Chemical Abstracts, Vol.99, No. 1, 4 July 1983, Columbus, Ohio (US),D.L. Dorset et al.:"Two-dimensional crystal packing of matrix porin.A channel forming protein in Escherichia coli outer membranes",see page 210, abstract 2009c & J. Mol. Biol. 1983, 165(2), 701-10
Chemical Abstracts, Vol. 98, No. 17, 25 April 1983, Columbus, Ohio (US), J. Jenkins et al.: "X-ray diffraction analysis of matrix porin, an integral membrane protein form Escherichia coli outer membranes", see page 218, abstract 139218u & J. Mol. Biol. 1983, 164 (2), 313-27

(73) Patentinhaber : Sleytr, Uwe B., Dipl.-Ing., Dr.
Pamhammerplatz 10
A-1170 Wien (AT)
Patentinhaber : Sara, Margit, Dipl.-Ing.
Vorgartenstrasse 90/2/24
A-1200 Wien (AT)

(72) Erfinder : Sleytr, Uwe B., Dipl.-Ing., Dr.
Pamhammerplatz 10
A-1170 Wien (AT)
Erfinder : Sara, Margit, Dipl.-Ing.
Vorgartenstrasse 90/2/24
A-1200 Wien (AT)

(74) Vertreter : Itze, Peter, Dipl.-Ing. et al
Patentanwälte Dipl.-Ing. Leopold Friebel
Dipl.-Ing. Peter Itze Amerlingstrasse 8
A-1060 Wien (AT)

## Beschreibung

Die Erfindung betrifft ein Verfahren zum Herstellen einer Struktur mit Membranen, die durchgehende Poren aufweisen, wobei die Membranen längs eines Kristallgitters angeordnete Proteinmoleküle oder proteinhältige Moleküle aufweisen, die aus Zellhullen von Mikroorganismen stammen, wobei die Membranen gegebenenfalls an oder in einem Träger an- bzw. eingebracht werden und wobei gegebenenfalls durch Behandlung mit mono- und/oder bifunktionalen Fremdmolekülen die Proteinmoleküle oder proteinhältigen Moleküle der Membran an ihren reaktiven Gruppen intramolekular und/oder miteinander und/oder mit dem Träger, gegebenenfalls kovalent, vernetzt werden.

Eine Struktur der vorgenannten Art ist aus der EP-A2-0 154 620 bekannt. Die zum Aufbau dieser Struktur verwendeten Membranen werden dabei vorteilhaft aus Proteinmolekülen oder proteinhältigen Molekülen, welche insbesondere von Zell-Hüllen von Prokaryonten erhalten wurden, durch einen als Selbstorganisation bezeichneten Rekristalliationsprozeß gebildet, danach z.B. auf oder in einem porösen Träger an- bzw. eingeschwemmt und schließlich einer chemischen Vernetzung unterworfen. Die wirksame Porenweite an den Membranen dieser Struktur liegt dabei insbesondere im Durchmesserbereich von 1 bis 200 nm (nanometer), vorteilhaft aber im Bereich von 1 bis 8 nm.

Bei Verwendung dieser Struktur als Ultrafiltrationsmembrane können scharfe Trennungen zwischen Molekülen mit geringem unterschiedlichen Molekulargewicht sowie höhere Durchflußraten erreicht werden als bei vorher bekannten Ultrafiltrationsmembranen. Der gewünschte Porendurchmesser wird dabei im wesentlichen durch die Auswahl des für die Membranherstellung verwendeten Mikroorganismus erreicht, deren Zell-Hüllen äußere Schichten, sogenannte S-Schichten (S-layers = Surface-layers) mit Poren mit in etwa dem angestrebten Porendurchmesser aufweisen. Dieser Porendurchmesser kann dabei noch durch Anlagerung von Fremdmolekülen an den Proteinmolekülen oder proteinhältigen Molekülen, die in den Porenbereich hineinreichen, variiert werden.

Der Erfindung liegt Aufgabe zugrunde, ein Verfahren der eingangs genannten Art zu schaffen, das weniger aufwendig ist als das bekannte Verfahren.

Erfindungsgemäß wird dies dadurch erreicht, daß die Zellen der Mikroorganismen, insbesondere Zellen von Prokaryonten, die zumindest eine Zellwandschicht aufweisen, in der die Proteinmoleküle oder die proteinhältigen Moleküle aneinanderliegend miteinander verbunden sind, wobei in dieser Zellwandschicht zwischen den längs eines Kristallgitters angeordneten Molekülen gemäß einem Gitter angeordnete Poren frei bleiben, gegebenenfalls aufgebrochen und die Zellhüllen in Fragmente unterteilt werden, und daß die nach Entfernen des Zellinhaltes sowie gegebenenfalls anderer Zellwand- oder Membranlipidschichten verbleibenden Zellhüllen oder Zellwandfragmente, welche aus der oder den Proteinmolekülen bestehenden Schicht(en) und gegebenenfalls den angrenzenden Zellwandschichten bestehen, an oder in den Träger an- oder eingebracht werden. Dabei kann vorteilhaft auch von Zell-Hüllen von Mikroorganismen ausgegangen werden, welche als Zellwandschichten zwei aus Proteinmolekülen oder proteinhältigen Molekülen aufgebaute Schichten und zwischen diesen, gegebenenfalls aus Peptidoglycan, Pseudomurein oder Zellulose bestehende Schichten aufweisen, wobei diese Zellwandschichten in Form von Zellwandfragmenten an oder in dem Träger an- bzw. eingebracht werden.

Gemäß einer vorteilhaften Ausgestaltung des erfindungsgemäßen Verfahrens wird von Zell-Hüllen von Mikroorganismen ausgegangen, in welchen die die Schichten formenden proteinhältigen Moleküle Glycoproteine sind.

Nach einer anderen vorteilhaften Ausgestaltung des erfindungsgemäßen Verfahrens werden die Mikroorganismenzellen osmotisch und/oder mechanisch, gegebenenfalls mit einer Zellmühle oder Ultraschall, und/oder durch Druckentspannung und/oder chemisch, gegebenenfalls mit einer French-Presse bzw. enzymatisch, aufgebrochen.

In weiteren vorteilhaften Ausgestaltungen des erfindungsgemäßen Verfahrens können als Träger

– eine poröse Schicht gegebenenfalls ein Mikrofilter eingesetzt werden und/oder

– eine Hilfsschicht eingesetzt werden, welche gegebenenfalls vor Verwendung der Membrane entfernt wird, oder

– Körper aus ionotropen Gelen verwendet herden, die von den aufzubringenden Zellwandfragmenten allseitig umschlossen werden.

Gemäß noch anderen vorteilhaften Ausgestaltungen des erfindungsgemäßen Verfahrens

– werden die Zellwandfragmente durch Aufsprühen und/oder Anschwemmen auf den Träger aufgebracht, und/oder es

– erfolgt das Aufbringen der Zellwandfragmente auf den Träger unter Druck- und oder Sogwirkung, oder es

– werden die Zellwandfragmente auf den Träger mechanisch aufgepreßt.

2

EP 0 207 100 B1

Nach einer weiteren Ausgestaltung des erfindungsgemäßen Verfahrens werden bei Verwendung von aus Polymeren bestehenden Trägern die Zellwandfragmente während des Polymerisations- oder Erstarrungsvorganges des Trägers in das sich porös ausbildende Trägermaterial eingebettet.

Gemäß noch einer anderen vorteilhaften Ausgestaltung des erfindungsgemäßen Verfahrens wird die Verbindung zwischen den Zellwandfragmenten und dem Träger durch eine, gegebenenfalls oberflächige Hitzebehandlung verstärkt.

Nach einer weiteren vorteilhaften Ausgestaltung der Erfindung ist das erfindungsgemäße Verfahren dadurch gekennzeichnet, daß die Behandlung mit mono- und/oder bifunktionellen Fremdmolekülen durch Aufbringen von gasförmigen und/oder flüssigen Vernetzungsmitteln erfolgt. Dabei kann die Behandlung mit den gasförmigen oder flüssigen Vernetzungsmitteln vorteilhaft zeitlich nacheinander erfolgen.

In einer anderen vorteilhaften Ausgestaltung des erfindungsgemäßen Verfahrens werden der Träger und/ oder die Zellwandfragmente vor Aufbringen der letzteren auf den Träger mit den Vernetzungsmitteln behandelt und diese erst nach dem Aufbringen der Zellwandfragmente auf dem Träger aktiviert.

Gemäß einer noch anderen vorteilhaften Ausgestaltung des erfindungsgemäßen Verfahrens werden vor dem Aufbringen der Zellwandfragmente auf den Träger für eine kovalente Vernetzung und/oder nebenvalente Bindung bestimmte reaktive Gruppen des Trägers und/oder der Moleküle der Zellwandfragmente freigelegt und/oder eingeführt.

Nach einer weiteren vorteilhaften Ausgestaltung des erfindungsgemäßen Verfahrens erfolgt das kovalente Vernetzen der Zellwandfragmente gleichzeitig mit dem kovalenten Vernetzen der Porteinmoleküle oder proteinhältigen Moleküle der aufgebrachten Zellwandfragmente.

In einer anderen vorteilhaften Ausgestaltung des erfindungsgemäßen Verfahrens werden vor, dem Aufbringen des bzw. der Vernetzungsmittel(s) die auf den Träger aufgebrachten Zellwandfragmente mittels einer porösen Schutzschicht abgedeckt, welche gegebenenfalls nach Beendigung des Vernetzungsvorganges entfernt wird.

Gemäß einer letzten vorteilhaften Ausgestaltung des erfindungsgemäßen Verfahrens schließlich werden die Zell-Hüllen nicht aufgebrochen, sondern die für den Aufbau der Struktur nicht benötigten Komponenten der Zellen, wie Cytroplasmamembran, Cytroplasma oder dlg. durch die Poren in den Zellhüllen herausgelöst.

Die Erfindung umfaßt schließlich folgende erfindungsgemäßen Verwendungen der nach dem vorstehenden erfindungsgemäßen Verfahren hergestellten Struktur, nämlich

– die Verwendung der Struktur als Ultrafilter, oder als Trennorgan für eine Gasseparation oder als Trennorgan für ein Ionenaustauschverfahren ;

– die Verwendung der Struktur als Träger für andere semipermeablen Membranen, die sich über Poren der Membranen der Strukur spannen, wobei gegebenenfalls diese anderen semipermeablen Membranen mit Proteinmolekülen oder proteinhältigen Molekülen der Membranen der Struktur über Carboxylgruppen und/oder Aminogruppen und/oder Sulfhydrylgruppen und/oder Hydroxylgruppen direkt oder über bifunktionelle Fremdmoleküle vernetzt sind. Dabei können diese anderen semipermeablen Membranen vorteilhaft sein : Hyperfiltrationsmembranen, gegebenenfalls Tensid- oder tensidartige Lipoid-Hyperfiltrationsmembranen, oder Trennorgane für eine Gasseparation oder Trennorgane für ein Ionenaustauschverfahren oder Trennorgane für ein Pervaporationsverfahren oder Lösungsdiffusionsmembranen,

die Verwendung als Trennmaterial für eine Durchdringungschromatographie, wobei die erzeugte Struktur aus nicht aufgebrochenen Zell-Hüllen besteht, aus der die nicht benötigten Komponenten herausgelöst und die verbleibenden Zell-Hüllen anschließend vernetzt wurden.

Gemäß einer weiteren erfindungsgemäßen Verwendung kann durch Ändern der Milieubedingungen eine Änderung der die Porenform und Porengröße bestimmenden Konformation der Moleküle der Membranen und-/oder eine Änderung der Ladungen an den Molekülen im Porenbereich der Membranen und damit eine Änderung der wirksamen Durchgangsweite der Membranen-Poren bewirkt werden. Dabei kann die Konformations- und/oder Ladungsänderung vorteilhaft durch Änderung des pH-Wertes, der Ionenstärke und/oder Zusammensetzung oder der Temperatur des die Struktur umgebenden Milieus und/oder vorteilhaft auch durch Wechsel der Art der flüssigen Phase bewirkt werden.

Eine weitere vorteilhafte Verwendung ist darin gelegen, daß durch Ändern der wirksamen Durchgangsweite der Poren einer Struktur Wertsubstanzen, wie Enzyme, pharmazeutische Wirkstoffe, Pestizide od.dgl. in der Struktur eingeschlossen und/oder diese Wertsubstanzen aus dieser Struktur ausgebracht werden.

Gemäß einer vorteilhaften Ausgestaltung dieser erfindungsgemäßen Verwendung wird eine Struktur eingesetzt, die eine die Wertsubstanzen einschließende Hülle bildet, deren Oberfläche zum Teil durch Membranen der Struktur belegt und zum Teil mit einer undurchlässigen Schicht versehen ist.

Nach einer anderen vorteilhaften Ausgestaltung der erfindungsgemäßen Verwendung wird eine Struktur eingesetzt, welche Membranen in Vesikelform aufweist.

3

Eine vorteilhafte Ausgestaltung dieser erfindungsgemäßen Verwendung ist ferner dadurch gekennzeichnet, daß zum Einbringen der Wertsubstanzen in die Struktur die Poren der vesikulären Membranen durch eine Milieuänderung erweitert werden, die Wertsubstanzen anschließend durch die erweiterten Poren in das Innere der Vesikel eingebracht und danach diese Poren durch eine weitere Milieuänderung wieder verkleinert werden.

Eine weitere vorteilhafte Verwendung einer erfindungsgemäß hergestellten Membrane kann darin gelegen sein, daß die Lösung bzw. Suspension der Proteinmoleküle oder proteinhältigen Moleküle oder Fragmente von Schichten aus solchen Molekülen in Kontakt mit einer Wertsubstanz gebracht wird, daß die Selbstorganisation der Proteinmoleküle oder proteinhältigen Moleküle längs der Oberfläche der Wertsubstanz erfolgt und daß – gegebenenfalls durch Behandlung mit mono- und/oder bifunktionellen Fremdmolekülen – die Moleküle dieser Membrane an reaktiven Gruppen substituiert und/oder über diese reaktiven Gruppen intramolekular und/oder miteinander kovalent vernetzt werden. Dabei kann auch vorteilhaft eine Wertsubstanz eingesetzt werden, die an oder in eine Trägersubstanz oder einen Trägerkörper gebunden bzw. eingebunden ist.

In dem nun folgenden Beispiel 1 seien nun vorteilhafte Ausführungswege eines neuen Verfahrens zum Herstellen von Strukturen beschrieben, die Membranen mit durchgehenden Poren aufweisen, nämlich Strukturen der Art, wie sie in der EP-A2-0154620 beschrieben sind, wobei bei diesen neuen Verfahren der Selbstorganisationsvorgang der Proteinmoleküle bzw. der proteinhältigen Moleküle und der aus diesen Molekülen aufgebauten Schichtfragmente zur Bildung größerer Membranen (in der EP-A2-0154620 als P-Membranen bezeichnet) vermieden wird.

Beispiel 1

Intakte Zellen von Bacillus stearothermophilus PV 72 mit einem Naßgewicht von 10 g werden in 40 ml TRIS-HCL-Puffer (50 mM, pH 7,2) suspendiert, und durch Einwirkung von Ultraschall aufgebrochen. Die Suspension wurde dabei zur Vermeidung autolytischer Prozesse in einem Eisbad gekühlt. Die Zellwandfragmente dieser Suspension wurden durch Zentrifugieren bei 20.000 g pelletiert und der untere Teil des Pellets, der aus noch intakten Zellen besteht, resuspendiert und nochmals einer Ultraschallbehandlung unterzogen. Die in Suspension vorliegenden Zellwandfragmente werden dann durch Zentrifugieren bei 20.000 g sedimentiert, resuspendiert und von ihnen die Cytroplasmamembran (eine Lipiddoppelschicht) mit 0,5% TRITON X-100 (in 50 mM TRIS-HCL-Puffer, pH 7,2) während 20 min bei 20°C desintegriert ("TRITON X-100" steht hier und in der Folge für Octylphenol-polyethylen-glycolether, einem milden Detergens), wonach die Zellwandfragmente mehrmals gewaschen werden. Die so gereinigten Zellwandfragmente bestehen nun aus der aus Glycoproteinmolekülen aufgebauten S-Schicht und der darunterliegenden Stützschicht aus Peptidoglycan. Die Größe der Flächenabmessungen dieser Zellwandfragmente wird elektronenmikroskopisch kontrolliert, sie sollen im Durchschnitt 500 nm betragen.

Mit Hilfe dieser Zellwandfragmente wird nun eine Ultrafiltrationsmembrane hergestellt, die im wesentlichen dieselben Eigenschaften besitzt wie die im Beispiel 2 der EP-A2-0154620 beschriebenen Ultrafiltrationsmembrane.

Als Trägermembrane für die herzustellende Ultrafiltrationsmembrane dient eine Nylonmikrofiltrationsmembrane der Fa. Pall, Cortland, N.Y., USA, Typ Ultripor $N_{66}$, mit einem mittleren Porendurchmesser von 100 nm. Das Trägermaterial besitzt freie Amino- und Carboxylgruppen im Verhältnis 1 : 1. Um zusätzliche reaktionsfähige Amino- und Carboxylgruppen freizusetzen, wird die einzusetzende Mikrofiltrationsmembrane bei 50°C während 60 min einer Behandlung mit Salzsäure (3,6 N) unterzogen, mit Aqua dest. gewaschen und in eine Ultrafiltrationseinrichtung – ähnlich der wie in Beispiel 1 und anhand von Fig. 5 der EP-A2-0154620 beschrieben – eingelegt.

Das Aufbringen der Zellwandfragmente auf den Träger und in die Matrix des Trägers erfolgt – analog wie gemäß den Beispielen 1 und 2 der EP-A2-0154620 – durch einen Anschwemmvorgang. Dazu werden, um eine möglichst gleichmässige Anschwemmung der Zellwandfragmente zu erzielen, 5 ml der Ausgangssuspension mit 50 ml Aqua dest. versetzt und in die Ultrafiltrationseinrichtung eingefüllt, wobei sich diese Suspension zunächst als Schicht über der eingelegten Mikrofiltrationsmembrane befindet. Über der Suspension wird nun durch Einleiten von Stickstoff ein Überdruck von $2.10^5$Pa aufgebracht, wodurch die flüssige Phase durch das Mikrofilter gepreßt und die Zellwandfragmente an oder in die Mikrofiltrationsmenbrane mit einer Belegung entsprechend einer Proteinmenge von 30 µg/cm² an- bzw. eingeschwemmt werden. Danach wird über einet im Oberteil der Ultrafiltrationseinrichtung angebrachten Düse mittels Preßluft von $3.10^5$Pa Überdruck als Vernetzungsmittel Glutardialdehyd (0,5% in 0,1 M Natrium-Cacodylat-Puffer, pH 7,0) fein verteilt auf die auf den Träger deponierten Zellwandfragmente aufgesprüht. Da das Vernetzungsmittel nach dem Aufsprühen auf die Zellwandfragmente durch den aufgebrachten Überdruck kontinuierlich durch die Zellwandfragmente und die Mikrofiltrationsmembrane gepreßt wird, bildet sich übet den Zellwandfragmenten kein stärkerer Flüssigkeitsfilm aus. Ein Vorteil dieser Art der Vernetzungsmittel-Aufbringung besteht darin, daß es in dem höchstens sehr dünnen Flüssigkeitsfilm kaum zu einer Wirbelbildung kommen kann, welche die auf dem Träger deponierten Zellwandfragmente bewegen und dabei ihre endgültige Fixierung stören könnte. Nach einer Sprüh- bzw. Vernetzungsdauer von 10 min wird die so hergestellte Ultrafiltrationsmembrane dreimal mit Aqua dest. gewaschen und die Trennkurve und die Durchflußrate – so wie in Beispiel 1 der EP-A2-0154620 beschrieben – bestimmt. Dabei zeigt sich, daß die Tatsache des Vorhandenseins der Peptidoglycan-Stützschicht auf das Trennverhalten der Ultrafiltrationsmembrane keinen Einfluß hat.

Gemäß einer vorteilhaften Variante dieses Beispiels werden als Ausgangsmaterial zur Herstellung einer Ultrafiltrationsmembrane intakte Zellen des Mikroorganismus Clostridium thermohydrosulfuricum (Stamm L111-69) verwendet. Intakte Zellen dieses Mikroorganismus werden zunächst in 50 mM TRIC-HCL-Puffer (pH 7,2) übergeführt und in einer French-Presse (French Pressure Cell Press) der firma American Instruments Corp., Silver Springs, Maryland, aufgebrochen. Dabei werden die in der Pufferlösung suspendierten Zellen unter hohem Druck komprimiert und erfahren dann beim Durchtritt durch eine Düse infolge der dabei eintretenden Druckentlastung eine plötzliche Expansion, die ein Zerreißen der Zellen zur Folge hat. Die so erhaltenen Zellwandfragmente werden durch dreimaliges Waschen mit der Pufferlösung von Zellinhaltsstoffen befreit und die Cytoplasmamembran durch Behandlung mit 1%igen TRITON X-100 in Aqua dest. während 30 min bei 37°C desintegriert. Die Zellwandfragmente werden dann von den Lipiden und anderen Verunreinigungen durch Zentrifugation getrennt und dann, analog wie in der ersten Variante dieses Beispiels beschrieben, weiterverarbeitet.

In der Folge werden einige vorteilhafte Varianten und Beispiele für einzelne Verfahrensschritte des erfindungsgemäßen Verfahrens angegeben.

für die Auswahl und Vorbereitung der Träger :

Die Verwendung von Nylonmikrofiltrationsmembranen, bei denen die hydrolytische Spaltung der Peptidbindung von Nylon durch Behandlung mit HCl oder mit N,N-Dimethylpropandiamin erfolgt um eine Erhöhung der Anzahl der freien Amino- und/oder Carboxylgruppen an Nylon zu erzielen. Beispiele für solche Nylonmikrofiltrationsmembranen sind z.B. folgende Typen der Fa. Pall, Cortland, N.Y., USA.
 – Pall Biodyne mit freien Amino- und Carboxylgruppen im Verhältnis 1 : 1 ;
 – Pall Aminodyne mit freien Aminogruppen ;
 – Pall Carboxydyne mit freien Carboxylgruppen.

Für das Aufbringen der Zellwandfragmente auf eine Trägermembrane :

Eine Suspension der Zellwandfragmente in $CH_3OH$ wird bei einer Temperatur von 50°C auf einem glatten Träger z.B. einer Glasplatte, aufgewalzt, wobei durch Verdampfen von $CH_3OH$ eine Eindickung der Suspension auftritt, bis schließlich eine feuchte Schicht mit einer Dicke von maximal 200 nm auf den Träger zurückbleibt. Der so beschichtete Träger wird in eine mit Formaldehyd gesättigten Atmosphäre gebracht und etwa 30 min inkubiert, wobei eine intra- und intermolekulare Vernetzung der Zellwandfragmente eintritt. Der durch die Vernetzung der Zellwandfragmente entstandene zusammenhängende dünne Film löst sich durch vorsichtiges Aufsprühen von destilliertem Wasser (4°C) von dem Träger und kann dann in einer feuchten Kammer auf einen stabilen, grobporigen Träger, z.B. ein Mikrofilter übertragen werden.

Für die mechanische Fixierung der Zellwandfragmente an oder in dem Träger :

Auf die aktive, das heißt mit Zellwandfragmenten belegte Seite einer Ultrafiltrationsmembrane – hergestellt wie in Beispiel 1 – wird ein Agar (2% in Aqua dest.) in flüssiger Form bei einer Temperatur von 45°C in einer Schichtdicke von 0,5-2 mm aufgegossen. Durch Abkühlen auf 20°C tritt eine Erstarrung des Agars in Form einer Gelschicht ein. Diese Gelschicht bildet nun eine Schutzschicht für die Ultrafiltrationsmembrane und dient gegebenenfalls als Vorfilter für größere Verunreinigungen, Sie kann jederzeit z.B. nach Abnutzung oder einer Verschmutzung der Gelporen wieder abgezogen und erneuert werden.

Gemäß einer anderen Variante werden zum Herstellen einer Ultrafiltrationsmembrane intra- und intermolekular vernetzte Zellwandfragmente mit einer Mischung aus Acrylamid, Bisacrylamid, Starter und Beschleuniger suspendiert, und die Mischung in Form einer Schicht bei 20°C polymerisieren und gelieren gelassen. Es bildet sich dabei ein poröser Träger aus, in dem die Zellwandfragmente eingebettet sind und der als Ultrafiltrationsmembrane verwendet werden kann.

Für die Bindung der Proteinmoleküle oder proteinhältigen Moleküle der Zellwandfragmente an den Träger :

Die freien Aminogruppen einer Trägermembrane werden mit Glutardialdehyd (5% in 0,1 M Na-Boratpuffer, pH 8,0) als Vernetzungsmittel aktiviert. Nach dem Anschwemmen der sich in Suspension befindlichen Zellwandfragmente an die Trägermembrane, z.B. durch die Sogwirkung eines (z.B. mit Hilfe einer Wasserstrahlpumpe erzeugten) Vakuums von 5-bis 6 10⁴Pa an der Unterdruckseite der Ultrafiltrationseinrichtung, tritt eine Bindung der Zellwandfragmente an die mit Glutardialdehyd aktivierten Aminogruppen der Trägermembrane ein. Die kovalente intra- und intermolekulare Vernetzung der Proteinmoleküle oder proteinhältigen Moleküle der Zellwandfragmente wird mit Hilfe von 2% Dimethylsuberimidat in 0,1 M Triethanolamin, pH 8,7, durchgeführt.

Gemäß einer anderen Variante werden die freien Carboxylgruppen der Trägermembrane mit EDC abgekürzt für 1-Ethyl-3-(Dimethylaminopropyl)Carbodiimid (0,1 M, pH 4,75) aktiviert. Nach dem Entfernen des überschüssigen Agens und Stoppen der Reaktion mit 0,01 M Na-Acetatpuffer, pH 4,75, wird mit Aqua dest. gewaschen. Die im Aqua dest. suspendierten Zellwandfragmente werden nach Zugabe von Hexamethylendiamin und 0,1 M EDC durch Anlegen eines Vakuums von 5.10⁴Pa an der Unterdruckseite der Ultrafiltrationseinrichtung an die Trägermembrane angeschwemmt. Die Proteinmoleküle oder proteinhältigen Moleküle der Zellwandfragmente werden über die durch das EDC aktivierten Carboxylgruppen über Hexanethylendiamin als Brückenbildner intra- und intermolekular vernetzt und kovalent an die Trägermembrane gebunden. Durch eine solche kovalente Bindung der Zellwandfragmente mit der Trägermembrane wird die Stabilität der Ultrafiltrationsmembrane wesentlich erhöht ; eine Abtragung der für die Ultrafiltration wesentlichen Zellwandfragmente durch mechanische Kräfte, wie sie z.B. in Cross-flow-Betrieb mit Anströmgeschwindigkeiten bis zu 7 m.s⁻¹ auftreten, wird damit unterbunden.

Für die Vernetzung :

Bei der Verwendung von Vernetzungsmitteln in flüssiger Phase muß für den Vernetzungsvorgang das Vernetzungsmittel durch die Poren der Zellwandfragmente gepreßt werden, wobei die Gefahr besteht, daß die erzeugte Strömung die auf oder in dem Träger auf- oder eingebrachten Zellwandfragmente bewegt, wodurch ihre endgültige Fixierung auf dem Träger erschwert wird. Um dies zu vermeiden, kann eine Vernetzung bzw. Vorvernetzung vorteilhaft auch durch ein gasförmiges Vernetzungsmittel vorgesehen werden.

Dazu wird als Träger eine Nylonmikrofiltrationsmembrane mit einer Porenweite von 100 nm in eine Ultrafiltrationseinrichtung eingesetzt. Die Zellwandfragmente werden durch Anlegen eines Vakuums von 5.10⁴Pa an der Unterdruckseite der Ultrafiltrationseinrichtung an oder in die Trägermembrane an- bzw. eingeschwemmt. In der Ultrafiltrationseinrichtung ist über der Trägermembrane ein Behälter mit Formaldehyd installiert. Für den Vernetzungsvorgang wird die Druckkammer der Ultrafiltrationseinrichtung zusammen mit dem Formaldehyd auf 80°C erwärmt. Der dabei verdampfende Formaldehyd, welcher bei dieser Temperatur neben seiner monomeren Form auch in oligomerer Form vorliegt, kommt nun mit den auf der Trägermembrane deponierten Zellwandfragmenten in Kontakt, wobei es insbesondere durch die oligomeren Formen des Formaldehyds, die wegen ihren größeren Kettenlängen größere Bereiche an den Oberflächen der Zellwandfragmente überspannen, zu einer intra- oder intermolekularen Vernetzung der Proteinmoleküle oder proteinhältigen Moleküle der Zellwandfragmente sowie zu deren Bindung an die Trägermembrane kommt. Die Anwendung eines gas- oder dampfförmigen Vernetzungsmittels hat den Vorteil, daß es hier zu keiner Wirbelbildung kommt, die gegebenenfalls eine Bewegung der deponierten Zellwandfragmente hervorrufen und so ihre endgültige Fixierung auf der Trägermembrane stören könnte. Nach einer Vernetzungszeit von 2 Stunden wird der restliche Formaldehyd mit Aqua dest. 1 Std. ausgewaschen.

Gemäß einer vorteilhaften Variante dieses Verfahrensschrittes kann zusätzlich und nach einer solchen Vernetzung mit Hilfe eines gas- oder dampfförmigen Vernetzungsmittels eine weitere Vernetzung mit flüssigen Vernetzungsmitteln stattfinden. Dazu werden diese flüssigen Vernetzungsmittel vorteilhaft auf die vorvernetzten Zellwandfragmente aufgetropft und durch deren Poren bzw. durch die poröse Trägermembrane hindurch durch ein an der Rückseite der Trägermembrane erzeugtes Vakuum abgesaugt. Dabei kommt es zu keiner mechanischen Beeinträchtigung der Zellwandfragmente. Als flüssige Vernetzungsmittel, die vorteilhaft nach einer Vorvernetzung durch Förmaldehyd eingesetzt werden, kommen z.B. Dimethylsuberimidat (Angriff auf die –NH₂-Gruppen im Protein) oder EDC (zur Vernetzung über –COOH-Gruppen im Protein) in Frage.

In dem nachstehenden Beispiel 2 wird eine Struktur mit vesikulären Membranen beschrieben, die aus Zell-Hüllen von Mikroorganismen hergestellt werden, ohne daß es zu einem Auffrechen der Zell-Hüllen kommt.

## Beispiel 2

Intakte Zellen des Mikroorganismus Bacillus stearothermophiles NRS 1536 (10 g Naßgewicht) werden in 50 ml 1% TRITON X-100 in 50 mM TRIS-HCl-Puffer (pH 7,2) suspendiert und die Suspension 30 min bei 37°C gerührt. TRITON X-100, ein mildes nicht ionisches Detergens, dringt infolge des Konzentrationsausgleiches durch die Poren der S-Schicht und der darunterliegenden zelleigenen, aus Peptidoglycan bestehenden Stützschicht in die Zelle ein und zerstört dort die an die Peptidoglycanschicht anschließende Cytoplasmamembran. Da die Cytoplasmamembran die hauptsächliche Diffusionsbarriere der Zell-Hülle darstellt, können nun nach deren Zerstörung die Inhaltsstoffe der Zelle nach außen diffundieren. Die Zellen werden nach der Behandlung mit TRITON X-100 bei 20.000 g zentrifugiert, zur Zerstörung von noch vorhandenen Cytoplasmamembranre- sten während 10 min bei 60°C einer weiteren Extraktion mit TRITON X-100 unterzogen, und neuerlich bei 20.000 g zentrifugiert. Die so präparierten Zellwandhüllen werden dann vier aufeinanderfolgenden Waschschritten unterzogen, die jeweils durch Rühren der Zellwandhüllen in Aqua dest. während 10 min und einer anschließenden Zentrifugation bei 20.000 g bestehen. Zum Abbau von noch vorhanderien Zellinhaltsstoffen wie Ribosomen und Nucleinsäuren werden einer Suspension der gewaschenen Zellwandhüllen in 40 ml Aqua dest. 2 mg Ribonuclease und 2 mg Desoxyribonuclease zugefügt, und 20 min bei 30°C unter Rühren inkubiert. Die Suspension wir dann bei 20.000 g zentrifugiert und die Zellen dann einigen Waschschritten mit Aqua dest. unterworfen.

Die so erhaltenen Zellwandhüllen werden nach Zentrifugation bei 20000 g in 0,1 M Triethanolamin (pH 8,5) resuspendiert, wonach – unter Rühren – zu 10 ml dieser Suspension 100 mg eines Vernetzungsmittels, z.B. Dunethylsuberimidat, zugeführt wird. Nach einer Reaktionsdauer von 60 min bei 20°C werden die vernetzten Zellwandhüllen durch Zentrifugation bei 20000 g gewnonnen, mit Aqua dest. gewaschen und können, ähnlich wie in Beispiel 2 erläutert, mit Enzymproteinen als Wertsubstanzen beladen werden.

In den nachfolgenden Beispielen 3 und 4 werden nun vorteilhafte Verwendungen der Änderung der Porenweite beschrieben.

## Beispiel 3

Bei diesem Beispiel kommt eine Ultrafiltrationsmembrane zur Anwendung, die auf Basis des Bacillus stearothermophilus PV 72 wie in Beispiel 1 der vorliegenden Beschreibung bzw. Beispiel 2 der EP-A2-0154620 beschrieben hergestellt ist. Es sollen nun die Proteine Carboanhydratase (30 kD), Ovalbumin (43 kD) und Glycerinaldehyd-3-phosphatdehydrogenase (140 kD) durch Filtrationsvorgänge getrennt werden.

Diese Proteine liegen zunächst in Aqua dest. gelöst vor.

In einem ersten Filtrationsschritt wird zunächst die Carbohydratase im Konzentrierungsbetrieb abgetrennt, wobei die Carbohydratase in das Permeat gelangt, wogegen Ovalbumin nahezu vollständig und Glycerinaldehyd-3-phosphatdehydrogenase zur Gänze im Retentat bleibt. Das Retentat wird danach von Aqua dest. in PBS umgepuffert. In der so umgepufferten Lösung, in der das Rückhaltevermögen der Ultrafiltrationsmembrane gegenüber Ovalbumin von etwa 90% auf etwa 25% herabgesetzt ist, wird das Ovalbumin dann in einem weiteren Diafiltrationsschritt ausgewaschen, wobei Glycerinaldehyd-3-phosphathydrogenase weiter im Retentat verbleibt und aus diesem dann gewonnen werden kann.

Die erfindungsgemäß hergestellte Ultrafiltrationsmembrane enthält also Membranen, die aus aneinanderliegenden Glycoproteinmolekülen aufgebaut sind, wobei jede der Membranporen von sechs angrenzenden Glycoproteinmolekülen begrenzt ist. Es wird nun vermutet, daß, analog wie bei in Lösung befindlichen Proteinmolekülen, welche nur in zwei Zuständen, nämlich ganz entfaltet und ganz gefalten bestehen, und sich durch eine Milieuänderung nur die Anzahl der sich in dem einen bzw. dem anderen Zustand befindlichen Moleküle ändert, die durch eine Milieuänderung bewirkte Änderung der Durchgangsweite der Membranporen auf einen ähnlichen Effekt beruht. Für die Konformation der Glycoproteine im Porenbereich und/oder die dort gebundenen Ladungen, besteht jeweils eine relativ kleine Zahl diskreter Zustände, deren Häufigkeiten von den Milieubedingungen, nämlich z.B. vom pH-Wert bzw. von der Ionenstärke abhängen. Dies könnte sich dann so auswirken, daß bei einer bestimmten Milieubedingung der größte Teil der Poren die kleinstmöglichste Porenweite und bei einer anderen Milieubedingung der größte Teil der Poren die größtmöglichste Porenweite aufweist. Für Filtrationen, zu deren Ergebnis nur die Poren der größten Poremweite wirksam sind, würde sich dann die wirksame Porendichte dieser größten Poren kontinuierlich mit den der Änderung der Milieubedingungen ändern.

Die nachstehend beschriebenen Filtrationsversuche bestätigen in Ergebnis diese Vorstellungen. Diese Versuche wurden jeweils in Aqua dest. sowie in verschiedenen Pufferlösungen, nämlich PBS (einer phosphatgepufferten physiologischen Kochsalzlösung), mit Sörensenpuffer (ein genau genormter Phosphatpuffer), mit 0,9% NaCl und mit 0,1 M CaCl$_2$ durchgeführt. Zur Erreichung eines Gleichgewichtszustandes mit der betreffenden Pufferlösung wurde die Ultrafiltrationsmembrane zunächst in dieser während 2 Stunden inkubiert. Die so vorbehandelten Ultrafiltrationsmembranen wurden dann für Filtrationsversuche in einer Ultrafiltrationseinrichtung, wie sie z.B. in Beispiel 1 anhand von Fig. 5 der obengenannten EP-A2-0154620 beschrieben ist, eingesetzt, und dabei – jeweils in derselben Pufferlösung wie für die Inkubation verwendet das Rückhaltevermögen (% R) des Filters gegenüber Ovalbumin und Rinderserumalbumin (in der Folge BSA genannt) bestimmt. Außerdem wurden mit denselben Substanzen Vergleichsversuche in Aqua dest. durchgeführt. Alle Versuche erfolgten mit einem Filtermembran-Überdruck von $2.10^5$Pa und einer Proteinkonzentration von 0,8 mg Protein pro ml einer 80%igen Einengung des Retentats sowie jeweils unter denselben Rührbedingungen (300 Umdr/min). Die Ergebnisse dieser Versuche sind in der nachstehenden Tabelle zusammengefaßt.

## Rückhaltevermögen in R(%)

| Milieu | Aqua dest | PBS | Sören-senp. | 0.9% NaCl | 0,1M CaCl$_2$ |
|---|---|---|---|---|---|
| pH | 5,5 | .7,2 | 6,8 | 5,5 | 5,5 |
| Ovalbumin (43kD) | 83-87 | 25-30 | 27 | 27 | 27 |
| BSA (66kD) | 95-97 | 52-56 | 57 | 53 | 57 |

Wie die Tabelle zeigt, gehen gegenüber den Verhältnissen bei Aqua dest. die Werte des Rückhaltevermögens bei den verwendeten Pufferlösungen stark zurück und zwar weitgehend unabhängig von dem eingesetzten Puffer. Dabei fällt auf, daß – wie der Vergleich der Werte der ersten Kolonne mit denen der zwei letzten Kolonnen zeigt – der Einfluß des pH-Wertes hier offenbar wesentlich geringer ist als der der Ionenstärke.

Ähnliche Versuche, die mit einer Ultrafiltrationsmembrane, die auf Basis der Bacillus stearothermophilus 3 c/NRs 1536 hergestellt wurde (siehe dazu Beispiel 1 der EP-A2-0154620), ergaben beim Austausch von Aqua dest. durch einen Puffer wie PBS und Sörensenpuffer wesentlich geringere Änderungen der Rückhaltevermögen. Sie betrugen hier nur etwa 7 bis 10%.

Die Auswahl der Mikroorganismen, deren S-Schichten bzw. die durch Rekristallisation der Proteinmoleküle oder proteinhältigen Moleküle dieser S-Schichten erzeugten Membranen (die in der Folge so wie in der EP-A2-0154620 als P-Membrane bezeichnet werden) bei einer Änderung der Milieubedingungen für den Anwendungszweck optimale Änderung der wirksamen Porenweite zeigen, erfolgt zweckmäßigerweise durch Überprüfung der Eigenschaften einer größeren Anzahl von Mikroorganismen.

Beispiel 4

In diesem Beispiel wird die Herstellung von vesikulären durchgehenden Poren aufweisende Membranen beschrieben sowie das Einbringen von Wertsubstanzen in diese Vesikel.

Man geht dabei von Zellen des Mikroorganismus Bacillus stearothermophilus (Stamm PV 72) aus, oder von Clostridium thermohydrosulfuricum (Stamm L111-69), dessen S-Schicht aus Glycoproteinen besteht und ein hexagonale Gitterstruktur (p6-Symmetrie) mit einer Periodizität von 14 nm und einer Porenweite in Aqua dest. von etwa 4 nm aufweist. 1 g Zellwandpräparationen werden mit 5 ml einer 5 M Guanidinhydrochloridlösung (in 50 mH TRIS-HCl-Puffer pH 7,2) 2 Stunden bei 25°C gerührt. "TRIS" steht hier und in der Folge als Abkürzung für Tris(hydroxymethyl)aminomethan. Dabei lösen sich von der Oberfläche der Zellwände Glycoproteinmoleküle der sogenannten S-Schichten. Die so extrahierten Zellwände werden durch Zentrifugation (1 Stunde bei 20.000 g) sedimentiert, wobei die Glycoproteinmoleküle in Überstand verbleiben. Das Guanidinhydrochlorid wird nun aus dem die Glycoproteinmoleküle enthaltenden Extrakt durch Dialyse gegen Aqua dest. (pH 5,5) oder 50 mM Tris-HCl-Puffer (pH 7,2) entfernt, wobei es durch Selbstorganisation der Glycoproteine zur Ausbildung von vesikulären Membranen kommt. Diese in Aqua dest. suspendierten Membranvesikel werden dann mit 2%igem Glutaraldehyd als Vernetzungsmittel (in 50 mM Phosphatpuffer, pH 7,2) bei 20°C während 2 Stunden behandelt. Dabei kommt es zu einer intra- bzw. intermolekularen Vernetzung der Glycoproteine. Nach dem Auswaschen des Vernetzungsmittels sind die Membranvesikel für eine Beladung mit Wertsubstanzen verwendbar. Dazu werden die Membranvesikel in einer Lösung einer Wertsubstanz, z.B. in einer Lösung von 10 mg/ml eines Proteins mit einem Molekulargewicht von 65 bis 70 kD in 0,9%iger Lösung von NaCl (pH 5,5) suspendiert. Unter diesen Milieubedingungen dringt die Wertsubstanz durch die nun erweiterten Poren- der Membranvesihel in diese ein, bis sich ein Konzentrationsausgleich einstellt. Nach Abzentrifugieren der Vesikel (z.B. 1 Stunde bei 15.000 g) werden diese in Aqua dest. (pH 5,5) resuspendiert. Durch diese gezielte Milieuveränderung von der NaCl-lösung zu Aqua dest. kommt es zu einer Verkleinerung der wirksamen Porenweite an Membranvesikeln derart, daß die Wertsubstanz nicht mehr wieder aus den Poren austreten kann und so in den Membranvesikeln festgehalten wird. Für das Freisetzen der Wertsubstanz aus den Membranvesikeln werden diese wieder in ein Milieu verbracht, dessen Ionenstärke etwa denselben Wert aufweist, der die Beladung der Membranvesikeln mit der Wertsubstanz ermöglicht hat.

Das erfindüngsgemäße Verfahren zum Ändern der wirksamen Porenweite einer Struktur, die Membranen mit durchgehenden Poren aufweist, kann auch vorteilhaft bei einer Struktur angewendet werden, die nach einem Verfahren hergestellt wurde, bei dem die Wertsubstanzen gleichzeitig mit dem Ausbilden dieser Struktur in- oder an diese ein- bzw. angebracht wurde. Das nachfolgende Beispiel 3 illustriert einen vorteilhaften Ausführungsweg dieses Verfahrens.

### Beispiel 5

Zum Fixieren eines die Wertsubstanz bildenden Enzymproteins an einem Träger, wird als Träger ein Polyacrylamidgel der Firma Biorad, Richmond, California, USA, das an seiner Porenoberfläche freie Carboxylgruppen aufweist, eingesetzt. Dieser Träger wird zunächst durch Dicyclohexylcarbodimid und N-Hydroxysuccinimid in einem wasserfreien Lösungsmittel wie Dioxan behandelt, wobei die Carboxylgruppen des Trägers aktiviert werden. Das lösungsmittel samt den überschüssigen Reagentien wird dann ausgewaschen und der Träger in 0,1 M Natriumphosphatpuffer (pH 7,8) übergeführt. Danach werden pro 1 g Träger 10 mg des Enzymproteins zugegeben und 5 Stunden bei 20°C inkubiert, wobei das Enzymprotein über die aktivierte Carboxylgruppe des Trägers kovalent an diesem gebunden wird.

Aus einer Zellwandpräparation des Mikroorganismus Clostridium thermohydrosulfuricum (Stamm L111-69) werden nach dem Ablösen der Glycoproteinmoleküle der S-Schicht dieses Mikroorganismus mit 5 M Guanidinhydrochlorid und Dialyse gegen aqua dest. P-Membranen hergestellt. 10 mg dieser P-Membranen werden dann durch Zugabe von 10 ml 0,01 M HCl in ihre Glycoproteinmoleküle zerlegt und diese extrahiert 10 ml dieses Extraktes werden mit 10 g des Trägers, an dem das Enzymprotein kovalent gebunden ist, vermischt und die Mischung bei 37°C gegen 10 mM $CaCl_2$-Lösung dialysiert. Durch die dadurch erzeugte pH-Wertänderung bilden sich durch Selbstorganisation der Glycoproteine an der äußeren Oberfläche des Gelkörpers Membranen aus, die den Gelkörper und das an ihm fixierte Enzym umhüllen. Die so gebildeten Membranen werden dann, analog wie in Beispiel 2 beschrieben, intra- bzw. intermolekular vernetzt.

Vorteilhafte Verwendungen der erfindungsgemäßen Verfahren und der durch ein erfindungsgemäßes Verfahren hergestellten Struktur, die gewerblich bedeutsam sind, sind in den vorstehend beschriebenen Beispielen erwähnt. Diese Strukturen, insbesondere solche, deren Herstellung anhand von Beispiel 1 samt dessen Varianten und von Beispiel 2 erläutert ist, können vorteilhaft in derselben Weise verwendet werden, wie es schon in der EP-A2-0154620 eingehend beschrieben wurde.

### Ansprüche

1. Verfahren zum Herstellen einer Struktur mit Membranen, die durchgehende Poren aufweisen, wobei die Membranen längs eines Kristallgitters angeordnete Proteinmoleküle oder proteinhältige Moleküle aufweisen, die as Zellhüllen von Mikroorganismen stammen, wobei die Membranen gegebenenfalls an oder in einem Träger an- bzw. eingebracht werden und wobei gegebenenfalls durch Behandlung mit mono- und/oder bifunktionalen Fremdmolekülen die Proteinmoleküle oder proteinhältigen Moleküle der Membran an ihren reaktiven Gruppen intramolekular und/oder miteinander und/oder mit dem Träger, gegebenenfalls kovalent, vernetzt werden, dadurch gekennzeichnet, daß die Zellen der Mikroorganismen, insbesondere Zellen von Prokaryonten, die zumindest eine Zellwandschicht aufweisen, in der die Proteinmoleküle oder die proteinhältigen Moleküle aneinanderliegend miteinander verbunden sind, wobei in dieser Zellwandschicht zwischen den längs eines kristallgitters angeordneten Molekülen gemäß einem Gitter angeordnete Poren frei bleiben, gegebenenfalls aufgebrochen und die Zellhüllen in Fragmente unterteilt werden, und daß die nach Entfernen des Zellinhaltes sowie gegebenenfalls anderer Zellwand- oder Membranlipidschichten verbleibenden Zellhüllen oder Zellwandfragmente, welche aus der oder den Proteinmolekülen bestehenden Schicht(en) und gegebenenfalls den angrenzenden Zellwandschichten bestehen, an oder in den Träger an- oder eingebracht werden.

2. Verfahren nach Anspruch 1, dadurch gekennzeichnet, daß von Zell-Hüllen von Mikroorganismen ausgegangen wird, welche als Zellwandschichten zwei as Proteinmolekülen oder proteinhältigen Molekülen aufgebaute kristalline Schichten und zwischen diesen, gegebenenfalls aus Peptidoglycan, Pseudomurein oder Zellulose bestehende Schichten aufweisen, daß diese Zellwandschichten in Form von Zellwandfragmenten an oder in dem Träger an- bzw. eingebracht werden.

3. Verfahren nach Anspruch 1 oder 2, dadurch gekennzeichnet, daß von Zell-Hüllen von Mikroorganismen ausgegangen wird, in welchen die die Schichten formenden proteinhältigen Moleküle Gycoproteine sind.

4. Verfahren nach einem der Ansprüche 1 bis 3, dadurch gekennzeichnet, daß die Mikroorganismenzellen osmotisch und/oder mechanisch, gegebenenfalls mit einer Zellmühle oder Ultraschall, und/oder durch Druckentspannung und/oder chemisch, gegebenenfalls mit einer French-Presse bzw. enzymatisch, aufgebrochen werden.

5. Verfahren nach einem der Ansprüche 1 bis 4, dadurch gekennzeichnet, daß als Träger eine Hilfsschicht eingesetzt wird, welche gegebenenfalls vor Verwendung der Membran entfernt wird.

6. Verfahren nach einem der Ansprüche 1 bis 5, dadurch gekennzeichnet, daß als Träger Körper aus ionotropen Gelen verwendet werden, die von den aufzubringenden Zellwandfragmenten allseitig umschlossen werden.

7. Verfahren nach einem der Ansprüche 1 bis 6, dadurch gekennzeichnet, daß die Zellwandfragmente durch Aufsprühen und/oder an sich bekanntes Anschwemmen auf den Träger aufgebracht werden.

8. Verfahren nach einem der Ansprüche 1 bis 7, dadurch gekennzeichnet, daß das Aufbringen der Zellwandfragmente auf den Träger unter Druck- und/oder Sogwirkung erfolgt.

9. Verfahren nach einem der Ansprüche 1 bis 6, dadurch gekennzeichnet, daß die Zellwandfragmente auf den Träger mechanisch aufgepreßt werden.

10. Verfahren nach einem der Ansprüche 1 bis 9, dadurch gekennzeichnet, daß die auf den Träger aufgebrachten Zellwandfragmente von einem weiteren Träger überlagert werden.

11. Verfahren nach einem der Ansprüche 1 bis 10, dadurch gekennzeichnet, daß bei Verwendung von aus Polymeren bestehenden Trägern die Zellwandfragmente während des Polymerisations- und/oder Erstarrungsvorganges des Trägers in das sich porös ausbildende Trägermaterial eingebettet werden.

12. Verfahren nach einem der Ansprüche 1 bis 10, dadurch gekennzeichnet, daß die Verbindung zwischen den Zellwandfragmenten und dem Träger durch eine gegebenenfalls oberflächige Hitzebehandlung verstärkt wird.

13. Verfahren nach einem der Ansprüche 1 bis 11, dadurch gekennzeichnet, daß die Behandlung mit mono- und/oder bifunktionellen Fremdmolekülen durch Aufbringen von gasförmigen und/oder flüssigen Vernetzungsmitteln erfolgt.

14. Verfahren nach Anspruch 13, dadurch gekennzeichnet, daß die Behandlung mit den gasförmigen oder flüssigen Vernetzungsmitteln zeitlich nacheinander erfolgt.

15. Verfahren nach Anspruch 12 oder 13, dadurch gekennzeichnet, daß der Träger und/oder die Zellwandfragmente vor Aufbringen der letzteren auf den Träger mit den Vernetzungsmitteln behandelt und diese erst nach dem Aufbringen der Zellwandfragmente auf dem Träger aktiviert werden.

16. Verfahren nach einem der Ansprüche 1 bis 15, dadurch gekennzeichnet, daß vor dem Aufbringen der Zellwandfragmente auf den Träger für eine kovalente Vernetzung und/oder nebenvalente Bindung bestimmte reaktive Gruppen des Trägers und/oder der Moleküle der Zellwandfragmente freigelegt und/oder eingeführt werden.

17. Verfahren nach einem der Ansprüche 13 bis 16, dadurch gekennzeichnet, daß das kovalente Vernetzen der Zellwandfragmente mit dem Träger gleichzeitig mit dem kovalenten Vernetzen der Proteinmoleküle oder proteinhältigen Moleküle der aufgebrachten Zellwandfragmente erfolgt.

18. Verfahren nach einem der Ansprüche 13 bis 17, dadurch gekennzeichnet, daß vor dem Aufbringen des bzw. der Vernetzungsmittel(s) die auf den Träger aufgebrachten Zellwandfragmente mittels einer porösen Schutzschicht abgedeckt werden, welche gegebenenfalls nach Beendigung des Vernetzungsvorganges entfernt wird.

19. Verfahren nach einem der Ansprüche 1, 13 und 14, dadurch gekennzeichnet, daß die Zell-Hüllen nicht aufgebrochen, sondern die für den Aufbau der Struktur nicht benötigten Komponenten der Zellen, wie Cytoplasmamembran, Cytoplasma oder dgl. durch die Poren in den Zellhüllen herausgelöst werden.

20. Verwendung einer gemäß einem Verfahren nach einem der Ansprüche 1 bis 19 hergestellten Struktur, dadurch gekennzeichnet, daß sie als Ultrafilter oder als Trennorgan für eine Gasseparation oder als Trennorgan für ein Ionenaustauschverfahren verwendet wird.

21. Verwendung einer gemäß einem Verfahren nach einem der Ansprüche 1 bis 19 hergestellten Struktur, dadurch gekennzeichnet, daß sie als Träger für andere semipermeable Membranen, die sich über Poren und-/oder Zellwandfragmente der Struktur spannen, verwendet wird, wobei gegebenenfalls diese anderen semipermeablen Membranen mit Proteinmolekülen oder proteinhältigen Molekülen der Zellwandfragmente der Struktur über Carboxylgruppen und/oder Aminogruppen und/oder Sulfhydrylgruppen und/oder Hydroxylgruppen direkt oder über bifunktionelle Fremdmoleküle vernetzt sind.

22. Verwendung nach Anspruch 21, dadurch gekennzeichnet, daß diese anderen semipermeablen Membranen Hyperfiltrationsmembranen, gegebenenfalls Tensid- oder tensidartige Lipoid-Hyperfiltrationsmembranen, sind.

23. Verwendung nach Anspruch 21, dadurch gekennzeichnet, daß diese anderen semipermeablen Membranen Trennorgane für eine Gasseparation sind.

24. Verwendung nach Anspruch 21, dadurch gekennzeichnet, daß diese anderen semipermeablen Membranen Trennorgane für ein Ionenaustauschverfahren sind.

25. Verwendung nach Anspruch 21, dadurch gekennzeichnet, daß diese anderen semipermeablen Membranen Trennorgane für ein Pervaporationsverfahren sind.

26. Verwendung nach Anspruch 21, dadurch gekennzeichnet, daß diese anderen semipermeablen Membranen Lösungsdiffusionsmembrane sind.

27. Verwendung einer gemäß dem Verfahren nach Anspruch 19 hergestellten Struktur, dadurch gekennzeichnet, daß sie als Trennmaterial für eine Druchdringungschromatographie verwendet wird.

28. Verwendung einer nach den Ansprüchen 1 bis 19 hergestellten Membran, dadurch gekennzeichnet, daß durch Änderung des Milieubedingungen eine Änderung der die Porenform und Porengröße bestimmenden Konformation der Moleküle der Membranen und/oder eine Änderung der Ladungen an den Molekülen im Porenbereich der Membranen und damit eine Änderung der wirksamen Durchgangsweite der Membranen-Poren bewirkt wird.

29. Verwendung nach Anspruch 28, dadurch gekennzeichnet, daß die Konformations- und/oder Ladungsänderung durch Änderung des pH-Wertes des die Struktur umgebenden Milieus bewirkt wird.

30. Verwendung nach Anspruch 28, dadurch gekennzeichnet, daß die Konformations- und/oder Ladungsänderung durch Änderung der Ionenstärke und/oder der Ionenzusammensetzung des die Struktur umgebenden Milieus bewirkt wird.

31. Verwendung nach Anspruch 28, dadurch gekennzeichnet, daß die Konformations- und/oder Ladungsänderung durch Änderung der Temperatur des die Struktur umgebenden Milieus bewirkt wird.

32. Verwendung nach Anspruch 28, dadurch gekennzeichnet, daß die Konformations- und/oder Ladungsänderung durch Wechsel der Art der flüssigen Phase bewirkt wird.

33. Verwendung nach einem der Ansprüche 28 bis 32, dadurch gekennzeichnet, daß durch Verändern der wirksamen Durchgangsweite der Poren einer Struktur Wertsubstanzen, wie Enzyme, pharmazeutische Wirkstoffe, Pestizide oder dgl. in der Struktur eingeschlossen und/oder diese Wertsubstanzen aus dieser Struktur ausgebracht werden.

34. Verwendung nach Anspruch 33, dadurch gekennzeichnet, daß eine Struktur eingesetzt wird, die eine die Wertsubstanzen einschließende Hülle bildet, deren Oberfläche zum Teil durch Membranen der Struktur belegt und zum Teil mit einer undurchlässigen Schicht versehen ist.

35. Verwendung nach Anspruch 33 oder 34, dadurch gekennzeichnet, daß eine Struktur eingesetzt wird, welche Membranen in Vesikelform aufweist.

36. Verwendung nach Anspruch 35, dadurch gekennzeichnet, daß zum Einbringen der Wertsubstanzen in die Struktur die Poren der vesikulären Membranen durch eine Milieuänderung erweitert werden, die Wertsubstanzen anschließend durch die erweiterten Poren in das Innere der Vesikel eingebracht und danach diese Poren durch eine weiter Milieuänderung wieder verkleinert werden.

37. Verwendung einer nach dem Verfahren gemäß einem der Ansprüche 1 bis 19 hergestellten Membran, dadurch gekennzeichnet, daß eine Lösung und/oder Suspension der eine Kristalline Schicht bildenden Partikel in Kontakt mit einer Wertsubstanz gebracht wird, daß die Selbstorganisation der proteinmoleküle oder proteinhältigen Moleküle längs der Oberfläche der Wertsubstanz erfolgt und daß – gegebenenfalls durch Behandlung mit mono- und/oder bifunktionellen Fremdmolekülen – die Moleküle dieser Membrane an reaktiven Gruppen substituiert und/oder über diese reaktiven Gruppen intramolekulär und/oder miteinander gegebenenfalls kovalent vernetzt werden.

38. Verwendung nach Anspruch 37, dadurch gekennzeichnet daß eine Wertsubstanz eingesetzt wird, die an oder in eine Trägersubstanz oder einen Trägerkörper gebunden bzw. eingebunden ist.

**Claims**

1. Method of producing a structure with membranes which have pores passing through them, the membranes having protein molecules or protein-containing molecules which are arranged along a crystal lattice and which are derived from cell envelopes of microorganisms, the membranes optionally being attached to or inserted into a carrier and the protein molecules or protein-containing molecules of the membrane being cross-linked, optionally covalently, at their reactive groups intramolecularly and/or with one another and/or with the carrier, optionally by treatment with mono- and/or bifunctional foreign molecules, characterised in that the cells of the microorganisms, particularly cells of prokaryotes, which have at least one cell wall layer in which the protein molecules or the protein-containing molecules are connected together adjacent to one another, whereby pores arranged in accordance with a lattice remain in this cell wall layer between the molecules arranged along a crystal lattice, are optionally broken up and the cell envelopes divided into fragments and that the cell envelopes or cell wall fragments, which remain after removal of the cell content and optionally of other cell wall or membrane lipid layers and which comprise the layer(s) comprising the protein molecules and optionally the adjoining cell wall layers, are attached to or inserted into the carrier.

2. Method as claimed in claim 1, characterised in that one starts with cell envelopes of microorganisms which have two crystalline layers composed of protein molecules or protein-containing molecules and between them layers optionally comprising peptidoglycan, pseudomurein or cellulose as the cell wall layers and that the cell wall layers are attached to or inserted into the carrier in the form of cell wall fragments.

3. Method as claimed in claim 1 or 2, characterised in that one starts with cell envelopes of microorganisms in which the protein-containing molecules forming the layers are glycoproteins.

4. Method as claimed in one of claims 1 to 3, characterised in that the microorganism cells are broken up osmotically and/or mechanically, optionally with a cell mill or ultrasound, and/or by pressure release and/or chemically, optionally with a French press or enzymatically.

5. Method as claimed in one of claims 1 to 4, characterised in that an auxiliary layer is used as the carrier which is optionally removed before use of the membrane.

6. Method as claimed in one of claims 1 to 5, characterised in that bodies of ionotropic gels are used as the carrier which are surrounded on all sides by the cell wall fragments to be applied.

7. Method as claimed in one of claims 1 to 6, characterised in that the cell wall fragments are applied to the carrier by spraying on and/or by deposition known per se.

8. Method as claimed in one of claims 1 to 7, characterised in that the application of the cell wall fragments to the carrier is effected under the action of pressure and/or suction.

9. Method as claimed in one of claims 1 to 6, characterised in that the cell wall fragments are mechanically pressed onto the carrier.

10. Method as claimed in one of claims 1 to 9, characterised in that the cell wall fragments applied to the carrier are overlain by a further carrier.

11. Method as claimed in one of claims 1 to 10, characterised in that when using carriers comprising polymers the cell wall fragments are embedded into the carrier material which forms in a porous manner during the polymerisation and/or solidification process of the carrier.

12. Method as claimed in one of claims 1 to 10, characterised in that the connection between the cell wall fragments and the carrier is reinforced by heat treatment, optionally of surface type.

13. Method as claimed in one of claims 1 to 11, characterised in that the treatment with mono- and/or bifunctional foreign molecules is effected by applying gaseous and/or liquid cross-linking agents.

14. Method as claimed in claim 13, characterised in that the treatment with the gaseous or liquid cross-linking agents is effected temporally after one another.

15. Method as claimed in claim 12 or 13, characterised in that the carrier and/or the cell wall fragments are treated before applying the latter to the carrier with the cross-linking agents and the latter are only activated after the application of the cell wall fragments to the carrier.

16. Method as claimed in one of claims 1 to 15, characterised in that, before the application of the cell wall fragments to the carrier, reactive groups of the carrier and/or of the molecules of the cell wall fragments intended for a covalent cross-linking and/or a non-covalent bonding are exposed and/or introduced.

17. Method as claimed in one of claims 13 to 16, characterised in that the covalent cross-linking of the cell wall fragments with the carrier occurs simultaneously with the covalent cross-linking of the protein molecules or protein-containing molecules of the applied cell wall fragments.

18. Method as claimed in one of claims 13 to 17, characterised in that before the application of the cross-linking agent(s) the cell wall fragments applied to the carrier are covered by means of a porous protective layer which is optionally removed after termination of the cross-linking process.

19. Method as claimed in one of claims 1, 13 and 14, characterised in that the cell envelopes are not broken up but instead the components of the cells which are not required for the formation of the structure, such as cytoplasm membranes, cytoplasm or the like, are eliminated through the pores in the cell walls.

20. Use of a structure produced by a method as claimed in one of claims 1 to 19, characterised in that it is used as an ultrafilter or as a separating element for gas separation or as a separating element for an ion exchange method.

21. Use of a structure produced by a method as claimed in one of claims 1 to 19, characterised in that it is used as a carrier for other semi-permeable membranes which extend over pores and/or cell wall fragments of the structure, these other semi-permeable membranes being optionally cross-linked directly or via bifunctional foreign molecules with protein molecules or protein-containing molecules of the cell wall fragments of the structure via carboxyl groups and/or amino groups and/or sulphydryl groups and/or hydroxyl groups.

22. Use as claimed in claim 21, characterised in that these other semi-permeable membranes are hyperfiltration membranes, optionally tenside or tenside-like lipoid hyperfiltration membranes.

23. Use as claimed in claim 21, characterised in that these other semi-permeable membranes are separating elements for gas separation.

24. Use as claimed in claim 21, characterised in that these other semi-permeable membranes are separating elements for an ion exchange method.

25. Use as claimed in claim 21, characterised in that these other semi-permeable membranes are separating elements for a pervaporation method.

26. Use as claimed in claim 21, characterised in that these other semi-permeable membranes are solution diffusion membranes.

27. Use of a structure produced by a method as claimed in claim 19, characterised in that it is used as a separating material for permeation chromatography.

28. Use of a membrane produced in accordance with claims 1 to 19, characterised in that a change of the conformation of the molecules of the membranes determining the pore shape and pore size and/or a change of the charges on the molecules in the pore region of the membranes and thus a change of the effective passage width of the membrane pores is effected by changing the environmental conditions.

29. Use as claimed in claim 28, characterised in that the change of conformation and/or charge is effected by changing the pH value of the environment surrounding the structure.

30. Use as claimed in claim 28, characterised in that the change in conformation and/or charge is effected by changing the ionic strength and/or the ionic composition of the environment surrounding the structure.

31. Use as claimed in claim 28, characterised in that the change in conformation and/or charge is effected by changing the temperature of the environment surrounding the structure.

32. Use as claimed in claim 28, characterised in that the change in conformation and/or charge is effected by changing the nature of the liquid phase.

33. Use as claimed in one of claims 28 to 32, characterised in that, by altering the effective passage width of the pores of a structure, valuable substances, such as enzymes, pharmaceutically active substances, pesticides or the like, are enclosed in the structure and/or these valuable substances are yielded from this structure.

34. Use as claimed in claim 33, characterised in that a structure is used which forms a shell enclosing the valuable substances whose surface is partly covered by membranes of the structure and is partly provided with an impermeable layer.

35. Use as claimed in claim 33 or 34, characterised in that a structure is used which has membranes in vesicular form.

36. Use as claimed in claim 35, characterised in that for the purpose of introducing the valuable substances into the structure the pores of the vesicular membranes are widened by a change in the environment, the valuable substances are subsequently introduced through the widened pores into the interior of the vesicles and thereafter these pores are made smaller again by a further change in the environment.

37. Use of a membrane produced by the method as claimed in one of claims 1 to 19, characterised in that a solution and/or suspension of the particles forming a crystalline layer are brought into contact with a valuable substance, that the self-organisation of the protein molecules or the protein-containing molecules occurs along the surface of the valuable substance and that – optionally by treatment with mono- and/or bifunctional foreign molecules – the molecules of this membrane are substituted at reactive groups and/or are cross-linked, optionally covalently, via these reactive groups intramolecularly and/or with one another.

38. Use as claimed in claim 37, characterised in that a valuable substance is used which is bonded to or incorporated into a carrier substance or a carrier body.

**Revendications**

1. Procédé pour fabriquer une structure comprenant des membranes qui présentent des pores traversants, dans laquelle les membranes présentent, le long d'un réseau cristallin, des molécules de protéines ou des molécules protéiques qui proviennent de l'enveloppe cellulaire de micro-organismes, structure dans laquelle les membranes sont éventuellement placées sur ou dans un support et dans laquelle, éventuellement par traitement avec des molécules étrangères mono- et/ou bifonctionnelles, les molécules de protéines ou les molécules protéiques de la membrane sont réticulées sur leurs groupes réactifs de façon intramoléculaire et/ou entre elles et/ou avec le support, éventuellement par covalence, caractérisé en ce que les cellules des micro-organismes, en particulier des cellules de procaryotes qui comportent au moins une couche de paroi cellulaire dans laquelle les molécules de protéines ou les molécules protéiques situées les unes à côté des autres sont reliées entre elles et dans laquelle des pores, disposés selon un réseau entre les molécules disposées le long d'un réseau cristallin, restent ouverts, sont éventuellement brisées et les enveloppes cellulaires sont divisées en fragments, et qu'après libération du contenu cellulaire ainsi qu'éventuellement d'autres couches de parois cellulaires ou de couches lipidiques de membrane, les enveloppes cellulaires ou les fragments de parois cellulaires restants, qui sont composés de la ou des couche(s) formée(s) de molécules de protéines et éventuellement des couches de parois cellulaires adjacentes, sont amenés sur ou dans le support.

2. Procédé selon la revendication 1, caractérisé en ce qu'on part d'enveloppes cellulaires de micro-organismes, lesquelles présentent, comme couches de parois cellulaires, deux couches cristallines constituées de molécules de protéines ou de molécules protéiques et, entre celles-ci, des couches formées éventuellement de peptidoglycane, de pseudomuréine ou de cellulose, et que ces couches de parois cellulaires, sous forme de fragments de parois cellulaires, sont amenées sur ou dans le support.

3. Procédé selon la revendication 1 ou 2, caractérisé en ce qu'on part d'enveloppes cellulaires de microorganismes dans lesquelles les molécules protéiques formant les couches sont des glycoprotéines.

4. Procédé selon l'une des revendications 1 à 3, caractérisé en ce que les cellules de micro-organismes sont brisées par rupture osmotique et/ou mécaniquement, éventuellement au moyen d'un broyeur de cellules ou par ultrasons, et/ou par relâchement de la pression et/ou chimiquement, éventuellement au moyen d'une presse à la française, ou de façon enzymatique.

5. Procédé selon l'une des revendications 1 à 4, caractérisé en ce qu'on utilise comme support une couche auxiliaire qu'on enlève éventuellement avant d'utiliser la membrane.

6. Procédé selon l'une des revendications 1 à 5, caractérisé en ce qu'on utilise comme support des corps à base de gels ionotropes qui sont entourés sur tous les côtés par les fragments de parois cellulaires à introduire.

7. Procédé selon l'une des revendications 1 à 6, caractérisé en ce que les fragments de parois cellulaires sont appliqués sur le support par pulvérisation ou par un procédé d'entraînement connu.

8. Procédé selon l'une des revendications 1 à 7, caractérisé en ce que l'application des fragments de parois cellulaires sur le support est réalisé sous l'action d'une poussée ou d'une aspiration.

9. Procédé selon l'une des revendications 1 à 6, caractérisé en ce que les fragments de parois cellulaires sont pressés mécaniquement sur le support.

10. Procédé selon l'une des revendications 1 à 9, caractérisé en ce que les fragments de parois cellulaires appliqués sur le support sont recouverts d'un autre support.

11. Procédé selon l'une des revendications 1 à 10, caractérisé en ce que, lorsqu'on utilise des supports composés de polymères, les fragments de parois cellulaires se logent dans la matière de support, qui se développe d'une manière poreuse, pendant l'opération de polymérisation et/ou de solidification.

12. Procédé selon l'une des revendications 1 à 10, caractérisé en ce que la liaison entre les fragments de parois cellulaires et le support est renforcée par un traitement thermique éventuellement en surface.

13. Procédé selon l'une des revendications 1 à 11, caractérisé en ce que le traitement au moyen de molécules étrangères mono- et/ou bifonctionnelles est réalisé par application d'agents de réticulation gazeux et/ou liquides.

14. Procédé selon la revendication 13, caractérisé en ce que les traitements au moyen des agents de réticulation gazeux ou liquides sont réalisés les uns après les autres.

15. Procédé selon la revendication 12 ou 13, caractérisé en ce qu'on traite le support et/ou les fragments de parois cellulaires avec les agents de réticulation avant d'appliquer lesdits fragments de parois cellulaires sur le support et on active les agents de réticulation seulement après avoir appliqué les fragments de parois cellulaires sur le support.

16. Procédé selon l'une des revendications 1 à 15, caractérisé en ce que, avant d'appliquer les fragments de parois cellulaires sur le support pour réaliser une réticulation par covalence et/ou une liaison par valence secondaire, on enlève et/ou introduit des groupes réactifs définis du support et/ou des molécules des fragments de parois cellulaires.

17. Procédé selon l'une des revendications 13 à 16, caractérisé en ce que la réticulation par covalence des fragments de parois cellulaires avec le support est réalisée en même temps que la réticulation par covalence des molécules de protéines ou des molécules protéiques des fragments de parois cellulaires appliqués.

18. Procédé selon l'une des revendications 13 à 17, caractérisé en ce que, avant d'appliquer le ou les agent(s) de réticulation, on recouvre les fragments de parois cellulaires, appliqués sur le support, au moyen d'une couche de protection poreuse, qu'on enlève éventuellement lorsque la phase de réticulation est terminée.

19. Procédé selon l'une des revendications 1, 13 et 14, caractérisé en ce que les enveloppes cellulaires ne sont pas brisées, mais les composants des cellules, qui ne sont pas nécessaires à la formation de la structure, comme la membrane cytoplasmique, le cytoplasme ou analogues, sont éliminés à travers les pores des enveloppes cellulaires.

20. Utilisation d'une structure préparée d'après le procédé selon l'une des revendications 1 à 19, caractérisée en ce qu'on utilise ladite structure comme ultrafiltre ou comme élément de séparation pour une séparation gazeuse, ou comme élément de séparation pour un procédé d'échange d'ions.

21. Utilisation d'une structure préparée d'après le procédé selon l'une des revendications 1 à 19, caractérisée en ce qu'on utilise ladite structure comme support pour d'autres membranes semi-perméables qui s'étendent au-dessus de pores et/ou de fragments de parois cellulaires de la structure, ces autres membranes semi-perméables étant éventuellement réticulées avec des molécules de protéines ou des molécules protéiques des fragments de parois cellulaires de la cellule au moyen de groupes carboxyle et/ou de groupes amino et/ou de groupes sulfhydryle et/ou de groupes hydroxyle, directement ou au moyen de molécules étrangères bifonctionnelles.

22. Utilisation selon la revendication 21, caractérisée en ce que ces autres membranes semi-perméables sont des membranes d'hyperfiltration, éventuellement des membranes d'hyperfiltration d'agents tensio-actifs ou de lipoïdes du type tensio-actifs.

23. Utilisation selon la revendication 21, caractérisée en ce que ces autres membranes semi-perméables sont des éléments de séparation pour une séparation gazeuse.

24. Utilisation selon la revendication 21, caractérisée en ce que ces autres membranes semi-perméables sont des éléments de séparation pour un procédé d'échange d'ions.

25. Utilisation selon la revendication 21, caractérisée en ce que ces autres membranes semi-perméables sont des éléments de séparation pour un procédé d'évaporation à travers une membrane.

26. Utilisation selon la revendication 21, caractérisée en ce que ces autres membranes semi-perméables sont des membranes de diffusion de solutions.

27. Utilisation d'une structure préparée d'après le procédé selon l'une des revendications 1 à 19, caractérisée en ce qu'on utilise ladite structure comme élément de séparation pour une chromatographie par percolation.

28. Utilisation d'une membrane préparée selon l'une des revendications 1 à 19, caractérisée en ce qu'une modification des conditions du milieu provoque une modification de la conformation des molécules des membranes, déterminant la forme et les dimensions des pores, et/ou une modification des charges des molécules dans la région des pores des membranes et, de ce fait, une modification du diamètre intérieur effectif des pores des membranes.

29. Utilisation selon la revendication 28, caractérisée en ce que la modification de la conformation et/ou des charges est réalisée par modification du pH du milieu ambiant de la structure.

30. Utilisation selon la revendication 28, caractérisée en ce que la modification de la conformation et/ou des charges est réalisée par modification de la force ionique et/ou de la composition ionique du milieu ambiant de la structure.

31. Utilisation selon la revendication 28, caractérisée en ce que la modification de la conformation et/ou des charges est réalisée par modification de la température du milieu ambiant de la structure.

32. Utilisation selon la revendication 28, caractérisée en ce que la modification de la conformation et/ou des charges est réalisée par remplacement du type de phase liquide.

33. Utilisation selon l'une des revendications 28 à 32, caractérisée en ce que, par modification du diamètre intérieur effectif des pores d'une structure, des substances précieuses, comme des enzymes, des substances actives pharmaceutiques, des pesticides ou analogues, sont enfermées dans la structure et/ou ces substances précieuses sont extraites de la structure.

34. Utilisation selon la revendication 33, caractérisée en ce qu'on met en oeuvre une structure qui forme une enveloppe enfermant les substances précieuses, dont la surface est en partie recouverte par des membranes de la structure et en partie pourvue d'une couche imperméable.

35. Utilisation selon la revendication 33 ou 34, caractérisée en ce qu'on met en oeuvre une structure qui présente des membranes en forme de vésicules.

36. Utilisation selon la revendication 35, caractérisée en ce que, pour introduire les substances précieuses dans la structure, on élargit les pores des membranes vésiculaires, par une modification du milieu ambiant, ensuite on introduit les substances précieuses à l'intérieur des vésicules, par les pores élargis, et après cela on réduit de nouveau la dimension des pores, par une nouvelle modification du milieu ambiant.

37. Utilisation d'une membrane préparée d'après le procédé selon l'une des revendications 1 à 19, caractérisée en ce qu'on met une solution et/ou une suspension des particules formant une couche cristalline en contact avec une substance précieuse, qu'il se produit une organisation automatique des molécules de protéines ou des molécules protéiques le long de la surface de la substance précieuse et que, éventuellement par un traitement au moyen de molécules étrangères mono- et/ou bifonctionnelles, les molécules de cette membrane sont substituées sur des groupes réactifs et/ou sont réticulées d'une manière intramoléculaire au moyen de ces groupes réactifs et/ou sont réticulées entre elles, éventuellement par covalence.

38. Utilisation selon la revendication 37, caractérisée en ce qu'on met en oeuvre une substance précieuse qui est fixée sur une substance de support ou sur un élément de support ou est intégrée à cette substance de support ou à cet élément de support.